(19) Europäisches Patentamt
European Patent Office
Office européen des brevets

(11) **EP 3 164 824 B1**

(12) **EUROPEAN PATENT SPECIFICATION**

(45) Date of publication and mention
of the grant of the patent:
**17.04.2024 Bulletin 2024/16**

(21) Application number: **15741950.8**

(22) Date of filing: **03.07.2015**

(51) International Patent Classification (IPC):
**G16H 50/30** *(2018.01)* **G16H 50/70** *(2018.01)*

(52) Cooperative Patent Classification (CPC):
**G16H 50/70; G16H 50/30**

(86) International application number:
**PCT/EP2015/065264**

(87) International publication number:
**WO 2016/001435 (07.01.2016 Gazette 2016/01)**

(54) **METHOD FOR PROGNOSING A RISK OF OCCURRENCE OF A DISEASE**

VERFAHREN ZUR BESTIMMUNG EINES MULTIFAKTORIELLEN WERTS DES RISIKOS DES AUFTRETENS EINER KRANKHEIT

PROCÉDÉ POUR DÉTERMINER UN SCORE MULTIFACTORIEL DE RISQUE D'APPARITION D'UNE MALADIE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **03.07.2014 EP 14306092**

(43) Date of publication of application:
**10.05.2017 Bulletin 2017/19**

(73) Proprietor: **Predilife**
**94805 Villejuif Cedex (FR)**

(72) Inventor: **RAGUSA, Stéphane**
**F-75007 Paris (FR)**

(74) Representative: **Vial, Lionel et al**
**Cabinet Lionel Vial**
**6 rue de Vaugondran**
**91190 Gif-sur-Yvette (FR)**

(56) References cited:
**EP-A1- 1 808 790 EP-A1- 2 278 510**

- **EMILIEN GAUTHIER ET AL: "Breast cancer risk score: a data mining approach to improve readability", PROCEEDINGS OF THE INTERNATIONAL CONFERENCE ON DATA MINING (DMIN), 2011, pages 15-21, XP055159067, Athens**

- **EMILIEN GAUTHIER ET AL: "Challenges to building a platform for a breast cancer risk score", RESEARCH CHALLENGES IN INFORMATION SCIENCE (RCIS), 2012 SIXTH INTERNATIONAL CONFERENCE ON, IEEE, 16 May 2012 (2012-05-16), pages 1-10, XP032201595, DOI: 10.1109/RCIS.2012.6240437 ISBN: 978-1-4577-1936-3**

- **Emilien Gauthier: "Evaluation du risque de maladie : conception d'un processus et d'un système d'information permettant la construction d'un score de risque adapté au contexte, application au cancer du sein", 2013, Artificial Intelligence. Télécom Bretagne, Université de Bretagne-Sud, XP055159072, abstract section 3.4 Application de notre processus sur l'exemple du cancer du sein chapter 5 Estimation du risque de cancer du sein avec l'algorithme des plus proches voisins**

- **PAWLOVSKY ALBERTO PALACIOS ET AL: "A method to select a good setting for the kNN algorithm when using it for breast cancer prognosis", IEEE-EMBS INTERNATIONAL CONFERENCE ON BIOMEDICAL AND HEALTH INFORMATICS (BHI), IEEE, June 2014 (2014-06), - 4 June 2014 (2014-06-04), pages 189-192, XP032625398, DOI: 10.1109/BHI.2014.6864336**

**Description**

Field of the invention

The present invention relates to a method for prognosing a risk of occurrence of a disease, such as breast cancer, in a subject.

Technical background

Breast cancer is the most common cancer among women worldwide, the second cause of cancer death among women in western countries after lung cancer, and a major growing public health problem in many countries. Current public health policies generally recommend biennial breast screening using mammogram, starting from age 40 or 50, age being the sole risk-factor considered for women to enter the programs. Mammographic screening has been associated on average with a 20% reduction in breast cancer specific mortality, though not without potential harms. Indeed, screening can lead to false-positive diagnoses, estimated at more than half of women after 10 years of annual screening, along with unnecessary biopsies. Women also face a risk of overdiagnosis and overtreatment, which is currently estimated around 11%, although estimates range between 1% and 30 %, depending on the populations and methods used. The women also face a small lifetime risk of radiation-induced cancer, estimated around 1.3-1.7 cases in 100 000 women for screen-film and digital mammography, and greater with other more recent breast imaging procedures.

While the current population-based strategy does have benefits, a more personalized screening strategy based on individual risk might improve the balance of benefits and harms for individuals and increase the efficiency of screening programs. With current programs, high-risk women who risk developing aggressive breast cancer early are not targeted, while low-risk women who will probably never develop breast cancer are frequently screened, facing potential harms. It appears therefore crucial that improved risk estimation models are brought to the clinic for better risk stratification, in view of implementing stratified screening policies.

Several models to predict breast cancer risk in the general population have been developed in the past 25 years, all of which use clinical variables based on family history, endogenous and exogenous hormonal exposure and previous diagnosis of benign breast disease. The most famous and widely used models are the Breast Cancer Risk Assessment Tool (BCRAT) (Gail et al. (1989) J Natl Cancer Inst, 81:1879-1886; Costantino et al. (1999) J Natl Cancer Inst, 91:1541-1548) and the Tyrer-Cuzick (IBIS) model (Tyrer et al. (2004) Stat Med 23:1111-1130). Most have only shown a modest discriminatory accuracy, with an area under the receiver operating characteristic (ROC) curve (AUC) around 0.60. The next generation, more recent, models have integrated breast mammographic density, which allowed an increase of the discriminatory accuracy, with AUC up to 0.66 (Tice et al. (2005) Breast Cancer Res Treat, 94:115-122; Barlow et al. (2006) J Natl Cancer Inst 98:1204-1214; Chen et al. (2006) J Natl Cancer Inst 98:1215-1226; Tice et al. (2008) Ann Intern Med 148:337-347). Gauthier et al. (2011) The international conference on data mining:15-21 disclose a k-nearest neighbour algorithm to compute a breast cancer risk score for different profiles from a public database.

Most risk prediction models, however, suffer from their opacity since they generally have the form of a parametric equation. Besides, it is difficult for clinicians to validate their accuracy in new populations without complicated validations processes, which could take numerous years. They have therefore been modestly translated into clinical use, and so far been limited to the purpose of high-risk identification, especially to identify women for cancer prevention trials.

It is therefore an object of the present invention to provide an alternative method for assessing multifactorial risk score, in particular in the frame of the prediction of the risk of occurrence of breast cancer, which could be easily understood by clinicians, and which could readily incorporate new risk-factors.

Description of the invention

The present invention follows from the unexpected finding by the inventors that a non-parametric k-nearest neighbor model they designed performed at least as well as the parametric Breast Cancer Surveillance Consortium (BCSC) 5-year risk model of the prior art (Tice et al. (2008) Ann. Intern. Med. 148:37-347 https://tools.bcsc-scc.org/BC5yearRisk/calculator.htm) for determining the five-year risk of occurrence of breast cancer in subjects with as few as five risk-factors taken into account, while being easy to understand by clinicians and readily amenable to incorporate new risk-factors. Advantageously, the need for less risk-factors and the simplicity of the method for determining the risk of occurrence of a disease of the invention favors an increased compliance of either clinicians or subjects to disease-risk assessment, and ultimately leads to health benefits by a better targeting of subjects at risk, thereby ensuring that subjects at high risk are given the appropriate prophylactic treatment or screening, while low-risk subjects are spared these measures and their potential side-effects.

Accordingly, the present invention relates to a method for determining a multifactorial score of risk of occurrence of a disease in a subject, comprising:

a) determining respective values of a plurality of disease risk-factors for the subject;

b) providing a database of individuals for whom the values of the plurality of disease risk-factors have been determined and the occurrence or not of the disease in the individuals is known;

c) transforming each value of the plurality of disease risk-factors of the subject and of the database individuals on a same disease incidence scale, wherein each value of disease risk-factor is transformed by the incidence of the disease in a population of individuals having the same said value of disease risk-factor;

d) selecting, within the database, a number of individuals which are at the lowest Euclidian distance of the subject with respect to other individuals of the database, wherein the Euclidian distance is based on the transformed values of the plurality of disease risk-factors and wherein the number of individuals which are at the lowest Euclidian distance of the subject is of from 10 individuals to 10000 individuals;

e) determining the ratio of the quantity of selected individuals in whom the disease has occurred to the number of selected individuals, thereby obtaining the multifactorial score of risk of occurrence of the disease in the subject.

As should be clear to one of skill in the art, the present invention can equivalently be defined as a method for determining, assessing or prognosing a risk, or the odds, of occurrence of a disease in a subject, wherein the risk, or the odds, of occurrence of the disease depend from a plurality of risk factors, comprising:

a) determining respective values of a plurality of disease risk-factors for the subject;

b) providing a database of individuals for whom the values of the plurality of disease risk-factors have been determined and the occurrence or not of the disease in the individuals is known;

c) transforming each value of the plurality of disease risk-factors of the subject and of the database individuals on a same disease incidence scale, wherein each value of disease risk-factor is transformed by the incidence of the disease in a population of individuals having the same said value of disease risk-factor;

d) selecting, within the database, a number of individuals which are at the lowest Euclidian distance of the subject with respect to other individuals of the database, wherein the Euclidian distance is based on the transformed values of the plurality of In a preferred embodiment of the invention, the above-defined method is a computer-implemented method.

Advantageously, the method of the invention allows calculating a distance between subjects or individuals being defined by several different qualitative or quantitative risk factors.

Also described is a method for the preventive or prophylactic treatment of a disease in a subject or for screening for a disease in a subject, comprising:

- determining a multifactorial score of risk of occurrence of a disease in a subject as defined above, or
- determining, assessing or prognosing the risk, or the odds, of occurrence of a disease in a subject as defined above, and

applying the preventive of prophylactic treatment, or screening for the disease if the score of risk is above a predetermined value or if the subject is at risk, or at odds, of being afflicted by the disease.

Also described is a method for determining if a subject may benefit from a preventive or prophylactic treatment of a disease, or from screening for a disease, comprising:

- determining a multifactorial score of risk of occurrence of a disease in a subject as defined above, or
- determining, assessing or prognosing the risk, or the odds, of occurrence of a disease in a subject as defined above, and

determining that the subject may benefit from a preventive or prophylactic treatment of the disease, or from screening for the disease, if the score of risk is above a predetermined value or if the subject is at risk, or at odds, of being afflicted by the disease.

The present invention also relates to a method for determining an estimated value of a biological factor in a subject for whom the respective values of a plurality of clinical factors have been determined comprising:

a) providing a database of individuals for whom the values of the plurality of clinical factors and of the biological factor, have been determined and the occurrence or not of the disease in the individuals is known;

b) transforming each value of the plurality of clinical factors of the subject and of the database individuals on a same disease incidence scale, wherein each value of clinical factors is transformed by the incidence of the disease in a population of individuals having the same said value of clinical factor;

c) selecting, within the database, a number of individuals which are at the lowest Euclidian distance of the subject with respect to other individuals of the database, wherein the Euclidian distance is based on the transformed values of the plurality of clinical factors and wherein the number of individuals which are at the lowest Euclidian distance of the subject is of from 10 individuals to 10000 individuals;

d) determining the average or median value of the biological factor of the selected individuals, thereby obtaining the estimated value of the biological factor of the subject.

Detailed description of the invention

The method of the invention relates to determining a multifactorial risk score of occurrence of a disease in a subject, i.e. the risk or the odds, that a subject will be afflicted by a disease, by taking into account several disease risk-factors of the disease. Generally, the risk score will be given for a time period, e.g. the risk score that the disease will occur in the next year or the next 2, 3, 4, 5, 10 or 20 years.

Any disease risk-factor, i.e. any characteristic or variable that can be linked to a subject or individual and which has been shown or is believed to be associated with an increased or decreased risk of occurrence of a disease according to the invention can be implemented in the method of the invention. However, it is preferred that at least one disease risk-factor according to the invention is selected in the group consisting of age, body mass, height, body mass index, a bodily dimension, occurrence of the disease in a relative, occurrence of a biopsy, occurrence or level of a biochemical marker, occurrence or level of an imaging marker, occurrence or level of a genetic marker, place of living or of working, and race or ethnicity. As intended herein, a "level" of a variable indicates both (i) the presence or absence of that variable and (ii) the value of that variable. The disease risk-factors according to the invention can either have numeric or qualitative values, i.e. the disease risk-factors can be quantitative or qualitative variables. Each subject can thus be defined by an array of coordinates, or a vector, consisting of the disease risk-factor values which have been determined. Preferably, at least one disease risk-factor according to the invention is qualitative.

As intended herein, a "clinical factor" is similar to a "disease-risk factor" according to the invention, that is any characteristic or variable that can be linked to a subject or individual and which has been shown or is believed to be associated with an increased or decreased risk of occurrence of a disease according to the invention, but it is preferred that it is a biological characteristic or variable of the subject or individual itself, such as age, body mass, height, body mass index, a bodily dimension, occurrence or level of a biochemical marker, occurrence or level of an imaging marker, occurrence or level of a genetic marker, and race or ethnicity. A "biological factor" is any characteristic or variable that can be linked to a subject or individual without it being necessarily associated with an increased or decreased risk of occurrence of a disease according to the invention. Preferably, the "biological factor" is a biological characteristic or variable of the subject or individual itself, such as age, body mass, height, body mass index, a bodily dimension, occurrence or level of a biochemical marker, occurrence or level of an imaging marker, occurrence or level of a genetic marker, and race or ethnicity.

According to the invention, there is provided, i.e. accessed, a database of individuals for which the values of the plurality of disease risk-factors have been determined, i.e. recorded, and the occurrence or not of the disease in the individuals is known. Preferably, the database will comprise at least 10000 individuals and at least 100 individuals in whom the disease has occurred. Preferably also, the database of the invention is a computer database.

According to the invention each value of disease risk-factor determined for the subject and for the database individuals is recoded on a same disease incidence scale. As intended herein, "recoded" is considered equivalent to "transformed". According to the invention the values of the different risk-factors are recoded on a same disease incidence scale, for instance from 0 to 1, from 0 to 100, from 0 to 10000 or from 0 to 100000, so as to be comparable between them. As intended herein a "disease incidence scale" is a numeric scale which is based on or is a function of the incidence of the disease in a population of individuals. As is well known to one of skill in the art, the incidence of a disease in a population of individuals is the proportion of individuals of the population who are newly afflicted by the disease in a given time period, also called follow up, i.e. the number of new disease cases within the population during the time period, i.e. during follow up. Typically, the time period is of 1 year, but longer time period, for example of 2, 3, 4, 5, 6, 7 8, 9 or 10 years are also within the frame of the present invention. Thus, preferably, each value of disease risk-factor is recoded by the incidence of the disease in a population of individuals having essentially the same said value of disease risk-factor, for instance expressed as the number of individuals afflicted by the disease within one year per 100000 individuals or within five years per 10000 individuals. It should be clear to one of skill in the art that the onset of follow up, i.e. the time period for determining the incidence of the disease, in a population of individuals having essentially the same said value of disease risk-factor is determined for each individual of said population and is the date at which said value of disease risk-factor was first recorded for the individual at the start of follow-up. In particular, the population of individuals having the same said value of disease risk factor may be selected among the database individuals. Besides, as should be clear to one of skill in the art, when selecting the population of individuals having the same said value of disease risk-factor, the values of the other diseases risk factors need not be taken into account, i.e. the recoding of a value of a given disease risk factor is performed by considering only the incidence of the disease associated to this specific value of said given disease risk factor. Preferably, the time period associated to the risk score according to the invention is identical to the time period associated to the incidence of the disease in a population of individuals according to the invention.

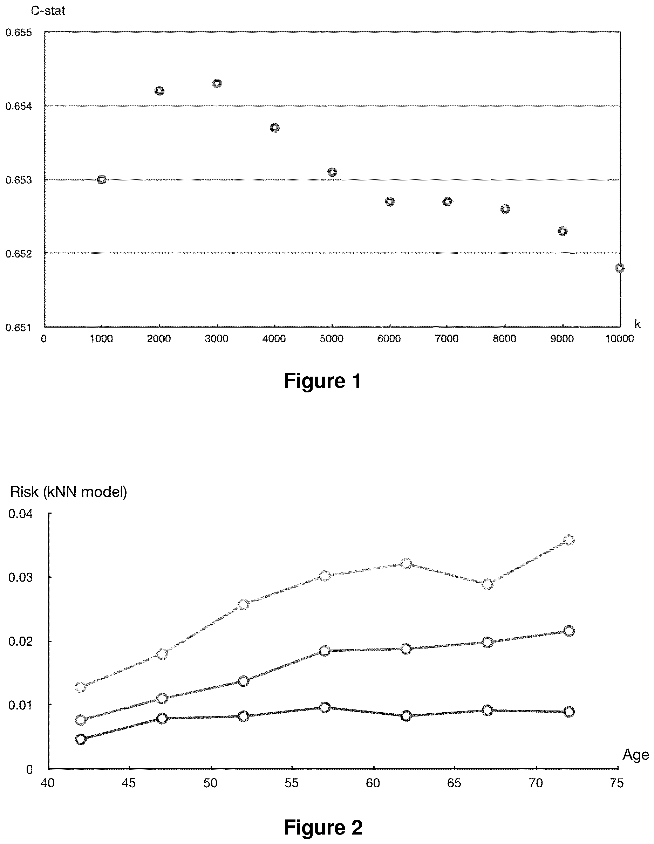

A number of individuals which are at the lowest Euclidian distance of the subject with respect to other individuals of the database are then selected within the database. These individuals form the "neighbors" of the subject according to the invention. The Euclidian distance is based on the recoded values of the plurality of disease risk-factors, i.e. each recoded value determined for the subject and the individuals of the database is taken into account for determining the Euclidian distance between the subject and each individual of the database. One of skill in the art can readily calculate the Euclidian distance of the invention. By way of example, the Euclidian distance between a subject A and an individual B respectively defined by their n recoded values ai and bi, (i varies from 1 to n), i.e. their coordinates in a n-dimension space, is $\sqrt{\sum_{i=1}^{n}(a_i - b_i)^2}$. The number of individuals which are at the lowest Euclidian distance of the subject according to the invention, denoted k in the following Example, can be easily determined by one skill in the art for a given database of individuals. By way of example, using a test cohort of subjects for whom the occurrence of the disease is known, one of skill in the art can implement the method of the invention with different numbers of individuals which are at the lowest Euclidian distance of the subject according to the invention, in order to select the number which yields the most accurate risk score, e.g. as is notably shown in **Figure 1.** Preferably, the number of individuals which are at the lowest Euclidian distance of the subject is of from 10 individuals to 10000 individuals, more preferably of from 2000 individuals to 6000 individuals, most preferably of about 3000 or 5000 individuals. Preferably also, the number of individuals which are at the lowest Euclidian distance of the subject is such that the quantity of selected individuals in whom the disease has occurred is of at least 10. Setting that such a minimum number of selected individuals in whom the disease has occurred is notably useful to preserve the anonymity of the individuals.

The multifactorial score of risk of occurrence of the disease in the subject, or the risk, or the odds, of occurrence of the disease in the subject, are then determined by calculating the ratio of the quantity of selected individuals in whom the disease has occurred to the number of selected individuals.

The method of the invention can be applied to any disease, including disorders, afflictions, adverse events (in particular death) or any pathological deviation from a healthy norm, which occurrence in a subject is multi-factorial, *i.e.* depends from a plurality of risk-factors. Accordingly, the method of the invention can be applied both to infectious and non-infectious diseases or afflictions. However, it is preferred that the disease is selected from the list consisting of a cancer, a neurodegenerative disease, a cardiovascular event, an autoimmune disease, allergy, an endocrine disorder, and osteoporosis. More particularly, it is preferred within the frame of the present invention that the disease is breast cancer.

Besides, where the disease is breast cancer, the at least one disease risk-factor is preferably selected in the group consisting of sex, age, occurrence of breast cancer in a relative, occurrence or level of a genetic marker, occurrence of a breast biopsy, previous occurrence of breast cancer, occurrence of radiotherapy to chest or face, occurrence of benign breast condition, race or ethnicity, body mass index, pregnancy history, breastfeeding history, menstrual history, usage of hormone replacement therapy, level of alcohol drinking, breast density, level of physical exercise, level of smoking, level of vitamin D, level of light exposure at night, level of exposure to diethylstilbestrol (DES), level of unhealthy food eating, level of eating grilled, barbecued or smoked food, level of exposure to chemicals in cosmetics, level of exposure to chemicals in food, level of exposure to chemicals for lawn and garden, level of exposure to chemicals in plastic, level of exposure to chemicals in sunscreen, and level of exposure to chemicals in water.

More preferably, where the disease is breast cancer, at least one disease risk-factor according to the invention is selected in the group consisting of age, occurrence of breast cancer in a relative, occurrence of a breast biopsy, race or ethnicity, and breast density.

Even more preferably, where the disease is breast cancer, the plurality of disease risk-factors according to the invention comprise at least age, occurrence of breast cancer in a relative, occurrence of a breast biopsy, race or ethnicity, and breast density.

Besides, breast density is preferably assessed in the frame of the present invention with the Breast Imaging-Reporting and Data System (BI-RADS).

The BI-RADS classification has been devised to standardize the reporting of the visual analysis of mammograms, i.e. X-Ray pictures of breasts, by radiologists in the frame of the assessment of breast cancer and breast cancer risk. The classification has then been extended to magnetic resonance imaging (MRI) and ultrasound pictures. The BI-RADS breast density categories are used to evaluate the density of non-pathogenic breasts:

> 1: Almost entirely fatty (i.e. non dense), which means that fibroglandular tissues make up less than 25% of the breast;
> 2: Scattered fibroglandular densities, which means that fibrous and glandular tissue makes up from 25 to 50% of the breast;
> 3: Heterogeneously dense, which means that the breast has more areas of fibrous and glandular tissue (from 51 to 75%) that are found throughout the breast;
4: Extremely dense, which means that the breast is made up of more than 75% fibroglandular tissue.

The subject and the individuals of the invention can be any type of living beings. However, it is preferred that the subject and the individuals of the invention are mammals, more preferably humans.

Besides, where the above-defined method is a computer-implemented method, the method can be run as a computer program, for instance on a personal computer or on a server and be accessed at distance through a terminal. The database can be uploaded on an internal memory of the computer or the server, such as random access memory (RAM), flash memory or hard disk drive (HDD), or be stored at distance on a second server.

Where the disease according to the invention is breast cancer, then it is preferred that the treatment according to the invention is chemotherapy, antibody therapy, radiation therapy or breast surgery, in particular breast ablation. In the frame of breast cancer it also preferred that the screening according to the invention is recurrent mammography, tomosynthesis, ultrasonography or Magnetic Resonance Imaging (MRI).

In addition, it is preferred that the above-defined methods of the invention are carried out by, or under the control or supervision of, a physician or doctor.

It is also preferred that the above-defined methods of the invention are not practiced on the human or animal body.

Preferably, the multifactorial score of risk is expressed as a number between 0 and 1, 0 being the lowest risk and 1 the highest risk (or certain occurrence), and the above-defined predetermined value is 0.1, 0.2, 0.3, 0.4, 0.5, 0.6, 0.7, 0.8 or 0.9, more preferably the above-defined predetermined value is equal to or above 0.5, 0.6, 0.7, 0.8 or 0.9. Similarly, it is preferred that the risk, or the odds, of occurrence of the disease according to the invention are expressed as a number between 0 and 1, 0 being the lowest risk and 1 the highest risk (or certain occurrence), and that a subject is considered at risk, or at odds, of being afflicted by the disease when the risk, or the odds, are equal to or above 0.1, 0.2, 0.3, 0.4, 0.5, 0.6, 0.7, 0.8 or 0.9, more preferably equal to or above 0.5, 0.6, 0.7, 0.8 or 0.9.

The invention will be further described with the following non-limiting figures and example.

Description of the figures

Figure 1
Figure 1 represents the value of the C-stat (vertical axis) for the kNN model of the invention as a function of the size of the neighborhood (k, horizontal axis, number of individuals).

Figure 2
Figure 2 represents the five-year risk level yielded by the method of the invention (vertical axis) by age category (horizontal axis, in years) for the lowest risk decile (bottom curve), the mean risk (middle curve) and the highest risk decile (top curve).

Figure 3

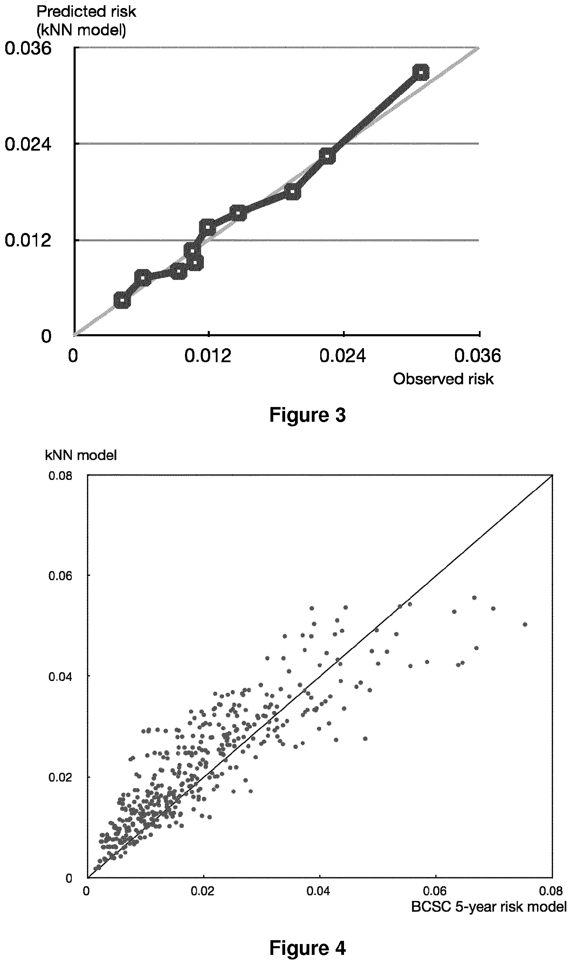

Figure 3 represents a reliability diagram showing the calibration by decile of risk level of the kNN model of the invention (vertical axis, predicted risk) versus the observed risk (horizontal axis, in percent).

Figure 4
Figure 4 represents the correlation of women's 5-year breast cancer risk between the BCSC 5-year risk model (vertical axis, in percent) and the kNN model of the invention (horizontal axis, in percent).

**EXAMPLE**

To test the method of the invention, its performance, discriminatory accuracy (overall and in sub-groups) and ability to classify women into clinically meaningful distinct risk groups were compared to those of the Breast Cancer Surveillance Consortium (BCSC) 5-year risk model built on the same data (Tice et al. (2008) Ann Intern Med 148:337-347). The correlation between the two models was evaluated.

**METHODS**

Study population

For the purpose of this study, the inventors used a subset of the data that were used for the construction and validation of the BCSC 5-year risk model (Tice et al. (2008) Ann Intern Med 148:337-347). Briefly, the original model used data from 1 095 484 women age 35 years or older who had at least 1 mammogram with breast density measured by using the Breast Imaging Reporting and Data System (BI-RADS) classification system in any of the seven mammography registries participating in the National Cancer Institute-funded Breast Cancer Surveillance Consortium (BCSC), as previously described in detail in Tice et al. (2008) Ann Intern Med 148:337-347. Women who had a diagnosis of breast cancer before their first eligible mammography examination were excluded, as well as those with cancer diagnosed within the first 6 months of follow-up (since the attempt is to predict longterm risk), and those with breast implants. Since the goal was to predict invasive cancer risk (as for the BCSC 5-year risk model), women in whom ductal carcinoma in situ was diagnosed were also excluded. When women had several mammograms, analysis was based on findings from the first mammogram. Age was separated into five-year groups. For the present invention, the inventors analyzed data from 629 229 women with complete follow-up information from 0.5 to 5.5 years from the index mammogram (a 5-year interval).

Measurement of risk-factors

Patient's characteristics, breast density, ascertainment of breast cancer cases and vital status were obtained from the BCSC database, and are described in detail in Tice et al. (2008) Ann Intern Med 148:337-347. Briefly, patient information was obtained primarily from self-report at the time of mammography, and included age, race/ethnicity, family history of breast cancer in a first-degree relative, and history of a breast biopsy. Ethnicity had been coded by using the expanded race and ethnicity definition currently used in the Surveillance, Epidemiology, and End Results (SEER) database and U.S. Vital Statistics. In the dataset, missing data for relatives with breast cancer and number of breast biopsies were set to 0, and women who selfidentified as mixed or other race were classified with those who did not report race and ethnicity. Breast density was assessed by community radiologists by using four BI-RADS categories at the time of mammographic interpretation ((ACR) ACoR. Breast Imaging Reporting and Data System Atlas (BI-RADS Atlas) 4. 4th ed. Reston, VA: American College of Radiology; 2003). Breast cancer outcomes (invasive cancer and ductal carcinoma in situ) were obtained at each site through linkage with the regional population-based SEER program, state tumor registries, and pathology databases. Vital status was obtained through linkage to SEER registries, state tumor registries, and the individual state vital statistics or the National Death Index.

Mathematical method and model development

In order to improve the readability of the risk model, the inventors used a modified version of the k-nearest-neighbors (kNN) algorithm (Fix & Hodges (1951) Discriminatory analysis, non parametric discrimination: consistency properties. Randolph Field, TX; Cover & Hart (1967) IEEE Transactions on Information Theory, 13:21-27). Accordingly, the method of the invention, also referred to hereafter as the kNN model, is a non-parametric method in which women's risk-factors are considered as a vector in a multi-dimensional space. The distance between two values of the same risk-factor (e.g. between two races, between breast density category 2 and 3, between 0 and 1 biopsy) needs to be quantified to be compared. To quantify this distance, the inventors have considered the difference in breast cancer incidence between the two values. For example, each value of race/ethnicity was coded with the observed average incidence (for example 0.0143 for White, 0.0124 for Black, and 0.0104 for Asian women). The same treatment was made for other risk-factors: age group (5-year groups), number of first-degree relatives with breast cancer (0-1-2 or more), number of breast biopsies (0-1 or more) and breast density (1 to 4). The process of enlargement of a neighborhood is made in a 5-dimensional space, since 5 risk-factors have been used in the present Example. The coding of each value of the 5 risk-factors and their distances are shown in **Table 1.** For example, the difference between 0 and 1 biopsy (237 - 116 = 121) can then be compared to the difference between density 3 and 2 (165 - 124 = 41). After this process, the distance between two women is simply the Euclidian distance.

**Table 1: Coding of each value of the parameters and their distances**

| Age category: | | |
|---|---|---|
| | Risk-factor value | incidence %/ 10 000 |
| | 42 | 73 |
| | 47 | 107 |
| | 52 | 138 |
| | 57 | 177 |
| | 62 | 187 |
| | 67 | 197 |
| | 72 | 217 |
| **Breast density:** | | |
| | Risk-factor value | incidence %/ 10 000 |
| | 1 | 75 |
| | 2 | 124 |
| | 3 | 165 |
| | 4 | 181 |
| **Race or ethnicity:** | | |
| | Risk-factor value | incidence %/ 10 000 |
| | 1 (White) | 143 |
| | 2 (Black) | 124 |
| | 3 (Asian) | 104 |
| | 8 (Hispanic) | 107 |
| | 9 (other/unknown) | 137 |
| **Breast biopsies:** | | |
| | Risk-factor value | incidence %/ 10 000 |
| | 0 | 116 |
| | 1 | 237 |
| **First-degree relatives with breast cancer:** | | |
| | Risk-factor value | incidence %/ 10 000 |
| | 0 | 132 |
| | 1 | 198 |
| | 2 | 244 |

The training set was then used to estimate the risk of each woman in the validation set. Firstly, a distance was calculated between the vector of risk-factors of the validation set woman and the vector of risk-factors of any women of the training set to define a neighborhood of a minimal size of k. Among all women, only the k women with the lower distances (i.e. the most similar women) were kept in the neighborhood. In the kNN model of the invention, a Euclidean distance was used to measure the similarity between women. For frequent profiles (e.g. White women with no family history), there were a sufficient number of women with the exact same profile. When insufficient, the closest women (i.e. those with the lowest Euclidian distances) were taken from the neighborhood. Most of the time, the enlargement concerned the neighboring age group, for which Euclidian distances are close **(Table 1)**. For race/ethnicity, the distance between a Black woman and a White woman is slightly lower than the distance between a Black woman and an Asian woman, as represented by the average breast cancer incidence. Thus, if the neighborhood of Black women did not have a sufficient number of people, it was enlarged by first including White women and not Asian women. To comply with BCSC rules on the protection of individual identity, the minimum number of breast cancer cases was set to 11 in a neighborhood.

Secondly, the risk level of the validation set was calculated as the ratio between the number of breast cancer cases in the neighborhood and the size of the neighborhood. This means that for each woman of the validation set, a distance is calculated with all women of the training set and the risk level is computed as the prevalence of breast cancer among women of the generated neighborhood.

Statistical analysis

The model's discriminatory accuracy, due to the continuous nature of the classifier according to the invention, was assessed with the Area Under the Roc Curve (AUC) (Egan (1975) Signal detection theory and ROC analysis. New York, NY: Series in Cognition and Perception Academic Press), also known as concordance statistic (c-statistic). The model discrimination was assessed by using a 5-fold crossvalidation method, through learning on 80% and testing on 20%. This step was done five times to cover the whole database, and the global AUC was the mean AUC.

Model calibration was assessed by calculating the ratio of expected breast cancer cases to observed breast cancer cases (E/O ratio) in the validation set. The best E/O ratio would be 1.0, meaning that the model predicts the same number of breast cancer cases as the actually observed number of breast cancer cases. To refine the calibration measurement, the E/O was computed by deciles of risk levels: in each decile, the mean score was computed and converted to an expected number of cases. The sum of the ten expected numbers of cases was then compared to the observed number of cases in the validation set using the E/O ratio. From the ten E/O ratios, a reliability diagram was deduced to analyze the calibration by decile of risk level.

Correlation with the BCSC 5-year risk model was assessed by computing the Pearson product-moment correlation coefficient as a measure of the linear dependence between the predicted risk level produced by the BCSC 5-year risk model and the method of the invention. A coefficient of 1.0 indicates strong positive linear correlation between predicted risk levels and a coefficient of 0.0 indicates no correlation. Correlation was computed with SAS 9.3.

## RESULTS

Population

As described before (Tice et al. (2008) Ann Intern Med 148:337-347), at the time of their earliest mammogram in the BCSC, 46% of women in this BCSC cohort were younger than age 50 years. The majority of women were white (71%), but more than 25 000 women represented each of the Black, Asian, and Hispanic groups. At a median follow-up of 5.3 years, 14 766 women had developed an invasive breast cancer.

Choice of the neighborhood size

The overall discrimination was measured for different values of k. The c-statistic was superior to 0.653 for k between 1000 and 5000 and the maximum was observed for k = 3000 women **(Figure 1).** The 5-year risk for women with minimal risk, aged 40, is around 0.6%, leading to around 18 cases in a neighborhood. In order to avoid sampling fluctuation and have rather 30 cases at least in these neighborhood, we choose a neighborhood size of 5000 women.

Overall performance and calibration of the method of the invention

The overall discrimination of the kNN model of the invention on the validation sample was good, with a c-statistic of 0.653 obtained on a 5-fold cross validation experiment. This appears very similar to the overall discrimination of c=0.658 of a 5-fold cross validation with the BCSC 5-year risk model. Overall calibration was very good, with an E/O ratio of 1.001 (obtained on the same 5 training/test repartitions) for the method of the invention, against an E/O ratio of 1.03 for the BCSC 5-year risk model.

Performance and calibration of the method of the invention in risk-factor subgroups

Discrimination and calibration of the kNN model of the invention were reasonably accurate across risk-factor subgroups **(Table 2)**. There was a slight overestimation of risk in women younger than 50 (1.00 to 1.02), while the BCSC 5-year risk model slightly underestimated the risk in this group (0.94 to 0.99). In women aged 65 or more, the kNN model of the invention was well calibrated (0.98 to 1.00), while the BCSC 5-year risk model overestimated the risk, up to 1.13 in the 65-69 category. The risk was slightly overestimated in both models for women with a breast density in category 1 or 2, whereas the risk is well calibrated for category 3 and 4 for the BCSC 5-year risk model and kNN model (1.02 and 1.00).

**Table 2 - Discrimination and calibration performances by subgroups**

| Risk-factor group | C-stat BCSC | C-stat kNN | E/O BCSC | E/O kNN |
|---|---|---|---|---|
| **Overall** | 0.658 | 0.653 | 1.03 | 1.001 |
| **Age** | | | | |
| 40-44 y | 0.63 | 0.63 | 0.94 | 1.02 |
| 45-49 y | 0.61 | 0.61 | 0.99 | 1.00 |
| 50-54 y | 0.62 | 0.62 | 0.96 | 1.01 |
| 55-59 y | 0.64 | 0.64 | 0.97 | 1.01 |
| 60-64 y | 0.63 | 0.63 | 1.04 | 1.00 |
| 65-69 y | 0.60 | 0.61 | 1.13 | 1.00 |
| 70-74 y | 0.61 | 0.61 | 1.08 | 0.98 |
| **Race or ethnicity** | | | | |
| White non hisp | 0.66 | 0.66 | 1.02 | 0.99 |
| Black non hisp | 0.63 | 0.63 | 1.00 | 1.12 |
| Asian | 0.66 | 0.67 | 0.95 | 1.08 |
| Hispanic | 0.67 | 0.67 | 0.94 | 1.02 |
| Other | 0.65 | 0.65 | 1.03 | 1.00 |
| **Bi-Rads density** | | | | |
| 1 | 0.67 | 0.67 | 1.04 | 1.08 |
| 2 | 0.64 | 0.64 | 1.02 | 1.02 |
| 3 | 0.65 | 0.65 | 1.00 | 1.00 |
| 4 | 0.64 | 0.64 | 1.00 | 0.93 |
| **Breast Biopsies** | | | | |
| 0 | 0.64 | 0.64 | 1.03 | 1.00 |
| >=1 | 0.62 | 0.62 | 0.99 | 1.00 |
| **First-degree relatives with breast cancer** | | | | |
| 0 | 0.65 | 0.65 | 0.99 | 1.00 |
| 1 | 0.64 | 0.64 | 1.07 | 1.00 |
| 2+ | 0.62 | 0.62 | 1.04 | 1.02 |

Correlation between the method of the invention and the Tice model

There was a very strong correlation between the kNN model of the invention and the Tice model, as shown in **Figure 1,** with a Pearson correlation coefficient measured at 0.94.

Identification of risk categories and concordance between models

The population was split into 5-year breast cancer risk deciles using the kNN model of the invention, and used to graphically illustrate an individual's risk assessment **(Figures 2 and 3)**. The median risk observed for a 40 year-old woman from the BCSC cohort is 0.64% at 5 years, and 1.29% for a 50 year-old woman. In the overall population, 2.9 out of 10 women have a 5-year risk beyond 1.66% and 1 out of 20 has a risk beyond 3%, whereas 10.5% of the women have a risk below the average observed risk at 40 years (0.64%).

There was a very strong concordance correlation between the kNN model and the BCSC 5-year risk model, as shown in **Figure 4,** with a concordance correlation coefficient measured at 0.96. A risk reclassification table using 1.66 and 3% thresholds **(Table 3)** shows that only 7.1% of women were non-concordantly classified between the BCSC 5-year risk model and kNN model.

**Table 3 - Concordance table using 1.66% and 3.00% threshold**

| | | BCSC 5-year risk model | | | |
|---|---|---|---|---|---|
| | | < 1.66% | 1.66% to 3.0% | > 3.0% | Total |
| kNN model | < 1.66% | 427 507 | 22 148 | 0 | 449 655 |
| | 1.66% to 3.0% | 8797 | 13 0170 | 10 108 | 149 075 |
| | > 3.0% | 62 | 3355 | 27 082 | 30 499 |
| | Total | 436 366 | 155 673 | 37 190 | 629 229 |

As a conclusion, the kNN model of the invention has potentials and advantages over previous models that make it a good candidate for future clinical use.

Firstly, the simple nearest neighbor approach enables easy integration of new biomarkers, without the need to re-create a new algorithm with every additional factor, as is the case with previous models. As the method of the invention is based on mean calculation, if a new factor is added, the neighborhood can change, but the principle remains the same. The model of the invention predicts a woman's risk by considering similar women - in the present Example, age, breast density, race/ethnicity, family history of breast cancer and history of breast biopsy have been considered, but a large range of potential factors may be taken into consideration in the future. In this regard, it is to be noted that with recent advances in genomic studies, principally Genome-Wide Association Studies (GWAS), an increasing number of polymorphisms associated with the risk of breast cancer have been identified (Varghese & Easton (2010) Curr Opin Genet Dev 20:201-209; Michailidou et al. (2013) Nat Genet 45:353-361; Sakoda et al. (2013) Nat Genet 45:345-348), with promising clinical utility for risk stratification and personalized screening strategies (Pashayan et al. (2011) Nat Genet 45:345-348). Moreover, a UK study has reported the feasibility of individual risk assessment using the Tyrer-Cuzick model, incorporating mammographic density and SNPs, in the context of a National Screening Program (Evans et al. (2012). Cancer Prev Res (Phila) 5:943-951).

Of note, SNPs can be added with the same neighborhood approach, since the Blast algorithm (Altschul et al. (1990) Nat Genet 45: 345-348) to match DNA sequences is also based on a distance evaluation between DNA sequences.

The flexibility of the method of the invention is of particular importance with common genetic variants that vary between ethnically distinct populations (Zheng et al. (2013) Hum Mol Genet 22:2539-2550). Besides, biomarkers and risk-factors that may be usefully included in risk prediction models will also come from ongoing research in the identification of new risk alleles using next generation sequencing of constitutional DNA (Hilbers et al. (2013) Clin Genet 84:407-414), epigenetic markers in peripheral blood (Brennan et al. (2012) Cancer Res 72:2304-2313; Xu et al. (2013) J Natl Cancer Inst 105:694-700), and metabolic alterations (Denkert et al. (2012) Genome Med 4:37).

Secondly, the approach of the invention is easily transposable from one population to another, enabling easier testing and shortening considerably the validation process. Although breast density is known to be a major risk-factor for breast cancer, the Gail model without breast density is still frequently used, because complex models such as the BCSC 5-year risk model have yet to be validated. The kNN model of the invention responds to the current need of a flexible model that is easy to validate.

Thirdly, its simplicity makes it more understandable and communicable to the public, which can be very useful for both clinicians and patients for clinical decisionmaking. The model is based on mean calculations, which is much easier to understand than models of the prior art, such as the BCSC 5-year risk model, which estimates 52 parameters on data, and yet showed equal performance.

In addition, the use of the model of the invention can contribute to the implementation of stratified screening policies. Thus, using the kNN model of the invention, 10.5% of the population may be considered low-risk, with a risk below the mean risk at age 40, among whom only 0.4% will actually develop breast cancer at 5 years (Negative Predictive Value (NPV) of 99.6%). This population might potentially be allowed to increase screening intervals. On the opposite, 29% have an increased risk (1.66% or more at 5 years) and 5.1% of the population a very high-risk (Positive Predictive Value (PPV) of 3.7%) and should be oriented to more sensitive screening tests and preventive interventions. From a public health perspective, this could lead to a better balance of benefits and harms of screening programs and more efficient cost distribution.

In summary, the inventors have developed a risk prediction method based on the nearest neighbor approach, which estimates a woman's 5-year risk for invasive breast cancer, with equal accuracy as the BCSC 5-year risk model of the prior art. The method of the invention has the advantages of being simple to use, easily applicable to other populations, and can easily integrate other risk-factors and biomarkers currently in development.

**Claims**

1.  A method for prognosing a risk of occurrence of a disease in a subject, comprising:

    a) determining respective values of a plurality of disease risk-factors for the subject;
    b) providing a database of individuals for whom the values of the plurality of disease risk-factors have been determined and the occurrence or not of the disease in the individuals is known;
    c) transforming each value of the plurality of disease risk-factors of the subject and of the database individuals on a same said disease incidence scale, wherein each value of disease risk-factor is transformed by the incidence of the disease in a population of individuals having the same said value of disease risk-factor;
    d) selecting, within the database, a number of individuals which are at the lowest Euclidian distance of the subject with respect to other individuals of the database, wherein the Euclidian distance is based on the transformed values of the plurality of disease risk-factors and wherein the number of individuals which are at the lowest Euclidian distance of the subject is of from 10 individuals to 10000 individuals;
    e) determining the ratio of the quantity of selected individuals in whom the disease has occurred to the number of selected individuals, thereby prognosing the risk of occurrence of the disease in the subject;

    wherein the method is a computer-implemented method.

2.  The method of claim 1, wherein at least one disease risk factor is qualitative.

3.  The method of any of claim 1 or 2, wherein the number of individuals which are at the lowest Euclidian distance of the subject is such that the quantity of selected individuals in whom the disease has occurred is of at least 10.

4.  The method of any of claims 1 to 3, wherein the disease is selected from the list consisting of a cancer, a neurodegenerative disease, a cardiovascular event, an autoimmune disease, allergy, an endocrine disorder, and osteoporosis.

5.  The method of any of claims 1 to 4, wherein the disease is breast cancer.

6.  The method of any of claims 1 to 5, wherein at least one disease risk-factor is selected in the group consisting of age, body mass, height, body mass index, a bodily dimension, occurrence of the disease in a relative, occurrence of a biopsy, occurrence or level of a biochemical marker, occurrence or level of an imaging marker, occurrence or level of a genetic marker, place of living or of working, race or ethnicity, level of physical exercise and occurrence or level of eating a food product.

7.  The method of any of claims 1 to 6, wherein the disease is breast cancer and at least one disease risk-factor is selected in the group consisting of sex, age, occurrence of breast cancer in a relative, occurrence or level of a genetic marker, occurrence of a breast biopsy, previous occurrence of breast cancer, occurrence of radiotherapy to chest or face, occurrence of benign breast condition, race or ethnicity, body mass index, pregnancy history, breastfeeding history, menstrual history, usage of hormone replacement therapy, level of alcohol drinking, breast density, level of physical exercise, level of smoking, level of vitamin D, level of light exposure at night, level of exposure to diethylstilbestrol (DES), level of unhealthy food eating, level of eating grilled, barbecued or smoked food, level of exposure to chemicals in cosmetics, level of exposure to chemicals in food, level of exposure to chemicals for lawn and garden, level of exposure to chemicals in plastic, level of exposure to chemicals in sunscreen, and level of exposure to chemicals in water.

8.  The method of any of claims 1 to 7, wherein the disease is breast cancer and at least one disease risk-factor is selected in the group consisting of age, occurrence of breast cancer in a relative, occurrence of a breast biopsy, race or ethnicity, and breast density.

9.  The method of any of claims 1 to 8, wherein the disease is breast cancer and the plurality of disease risk-factors comprise at least age, occurrence of breast cancer in a relative, occurrence of a breast biopsy, race or ethnicity, and breast density.

10. The method of any of claims 7 to 9, wherein breast density is assessed with the Breast Imaging-Reporting and Data System (BI-RADS).

11. A method for determining an estimated value of a biological factor in a subject for whom the respective values of a plurality of clinical factors have been determined, comprising:

> a) providing a database of individuals for whom the values of the plurality of clinical factors and of the biological factor, have been determined and the occurrence or not of a disease in the individuals is known;
> b) transforming each value of the plurality of clinical factors of the subject and of the database individuals on a same disease incidence scale wherein each value of clinical factors is transformed by the incidence of the disease in a population of individuals having the same said value of clinical factor;
> c) selecting, within the database, a number of individuals which are at the lowest Euclidian distance of the subject with respect to other individuals of the database, wherein the Euclidian distance is based on the transformed values of the plurality of clinical factors and wherein the number of individuals which are at the lowest Euclidian distance of the subject is of from 10 individuals to 10000 individuals;
> d) determining the average or median value of the biological factor of the selected individuals, thereby obtaining the estimated value of the biological factor of the subject.

**Patentansprüche**

1. Verfahren zur Risikoprognose des Auftretens einer Krankheit bei einer Person, umfassend:

> a) Bestimmung des jeweiligen Wertes mehrerer Krankheitsrisikofaktoren bei der Person,
> b) Bereitstellung einer Datenbank von Individuen, bei denen die Werte der mehreren Krankheitsrisikofaktoren bestimmt wurden und das Auftreten oder Nichtauftreten der Krankheit bei den Individuen bekannt ist,
> c) Umformen jeden Wertes der mehreren Krankheitsrisikofaktoren der Person und der Datenbankindividuen in eine und dieselbe Erkrankungsskala der genannten Krankheit, wobei jeder Wert eines Krankheitsrisikofaktors mit dem Auftreten der Krankheit in einer Population von Individuen, die denselben genannten Wert des Krankheitsrisikofaktors aufweisen, umgeformt wird,
> d) Auswahl einer Anzahl von Individuen innerhalb der Datenbank, die sich im Vergleich zu anderen Individuen in der Datenbank im niedrigsten euklidischen Abstand zur Person befinden, wobei der euklidische Abstand auf den umgeformten Werten der mehreren Krankheitsrisikofaktoren beruht und wobei die Anzahl an Individuen, die sich im niedrigsten euklidischen Abstand zur Person befinden, zwischen 10 Individuen und 10000 Individuen beträgt,
> e) Bestimmung des Verhältnisses der Anzahl ausgewählter Individuen, bei denen die Krankheit aufgetreten ist, zur Anzahl ausgewählter Individuen, womit das Risiko des Auftretens der Krankheit bei der Person prognostiziert wird,

> wobei das Verfahren ein Computer-implementiertes Verfahren ist.

2. Verfahren nach Patentanspruch 1, in dem mindestens einer der Krankheitsrisikofaktoren qualitativ ist.

3. Verfahren nach irgendeinem der Patentansprüche 1 oder 2, in dem die Anzahl der Individuen, die sich im niedrigsten euklidischen Abstand zur Person befinden, derart ist, dass die Anzahl ausgewählter Individuen, bei denen die Krankheit aufgetreten ist, mindestens 10 beträgt.

4. Verfahren nach irgendeinem der Patentansprüche 1 bis 3, in dem die Krankheit aus der Liste gewählt wird, bestehend aus Krebs, neurodegenerativen Krankheiten, Herz-Kreislauferkrankungen, Autoimmunkrankheiten, Allergien, endokrinologischen Störungen und Osteoporose.

5. Verfahren nach irgendeinem der Patentansprüche 1 bis 4, in dem die Krankheit Brustkrebs ist.

6. Verfahren nach irgendeinem der Patentansprüche 1 bis 5, in dem mindestens ein Krankheitsrisikofaktor aus der Gruppe gewählt wird, bestehend aus Alter, Körpergewicht, Größe, Body-Mass-Index, eine Körperabmessung, Auftreten der Krankheit bei einem Verwandten, Ausführung einer Biopsie, Auftreten oder Betrag eines biochemischen Markers, Auftreten oder Betrag eines Bildmarkers, Auftreten oder Betrag eines genetischen Markers, Lebens- oder Arbeitsort, Rasse oder Volkszugehörigkeit, Grad der körperlichen Betätigung und Auftreten oder Umfang des Verzehrs eines Nahrungsmittels.

7. Verfahren nach irgendeinem der Patentansprüche 1 bis 6, in dem die Krankheit Brustkrebs ist und mindestens ein Krankheitsrisikofaktor aus der Gruppe gewählt wird, bestehend aus Geschlecht, Alter, Auftreten von Brustkrebs bei einem Verwandten, Auftreten oder Betrag eines genetischen Markers, Ausführung einer Brustbiopsie, vorheriges Auftreten von Brustkrebs, Ausführung von Strahlentherapie von Brust oder Gesicht, Auftreten von gutartigen Brustveränderungen, Rasse oder Volkszugehörigkeit, Body-Mass-Index, Schwangerschaftsvorgeschichte, Stillvorgeschichte, Menstruationsvorgeschichte, Ausführung einer Hormonersatztherapie, Niveau des Alkoholkonsums, Brustdichte, Grad der körperlichen Betätigung, Umfang des Rauchens, Vitamin-D-Niveau, Umfang der Lichtexposition bei Nacht, Umfang der Exposition gegenüber Diethylstilbestrol (DES), Umfang der Aufnahme ungesunder Nahrungsmittel, Umfang der Aufnahme gegrillter oder geräucherter Speisen, Umfang der Exposition gegenüber Chemikalien in Kosmetika, Umfang der Exposition gegenüber Chemikalien in der Nahrung, Umfang der Exposition gegenüber Chemikalien für Rasen und Garten, Umfang der Exposition gegenüber Chemikalien in Kunststoff, Umfang der Exposition gegenüber Chemikalien in Sonnencremes, und Umfang der Exposition gegenüber Chemikalien in Wasser.

8. Verfahren nach irgendeinem der Patentansprüche 1 bis 7, in dem die Krankheit Brustkrebs ist und mindestens ein Krankheitsrisikofaktor aus der Gruppe gewählt wird, bestehend aus Alter, Auftreten von Brustkrebs bei einem Verwandten, Ausführung einer Brustbiopsie, Rasse oder Volkszugehörigkeit, und Brustdichte.

9. Verfahren nach irgendeinem der Patentansprüche 1 bis 8, in dem die Krankheit Brustkrebs ist und die mehreren Krankheitsrisikofaktoren mindestens Alter, Auftreten von Brustkrebs bei einem Verwandten, Ausführung einer Brustbiopsie, Rasse oder Volkszugehörigkeit, und Brustdichte umfassen.

10. Verfahren nach irgendeinem der Patentansprüche 7 bis 9, in dem die Brustdichte nach dem Breast Imaging-Reporting and Data System (BI-RADS) erfasst wird.

11. Verfahren zur Bestimmung eines Schätzwertes eines biologischen Faktors bei einer Person, bei der die entsprechenden Werte mehrerer klinischer Faktoren bestimmt wurden, umfassend:

a) Bereitstellung einer Datenbank von Individuen, bei denen die Werte der mehreren klinischen Faktoren und des biologischen Faktors bestimmt wurden und das Auftreten oder Nichtauftreten einer Krankheit bei den Individuen bekannt ist,
b) Umformen jeden Wertes der mehreren klinischen Faktoren der Person und der Datenbankindividuen in eine und dieselbe Erkrankungsskala, wobei jeder Wert der klinischen Faktoren mit dem Auftreten der Krankheit in einer Population von Individuen, die denselben genannten Wert des klinischen Faktors aufweisen, umgeformt wird,
c) Auswahl einer Anzahl von Individuen innerhalb der Datenbank, die sich im Vergleich zu anderen Individuen in der Datenbank im niedrigsten euklidischen Abstand zur Person befinden, wobei der euklidische Abstand auf den umgeformten Werten der mehreren Krankheitsrisikofaktoren beruht und wobei die Anzahl an Individuen, die sich im niedrigsten euklidischen Abstand zur Person befinden, zwischen 10 Individuen und 10000 Individuen beträgt,
d) Bestimmung des Mittel- oder Medianwertes des biologischen Faktors der ausgewählten Individuen, um damit den Schätzwert des biologischen Faktors bei der Person zu erhalten.

**Revendications**

1. Méthode de pronostic du risque d'apparition d'une maladie chez un sujet, comprenant :

a) déterminer les valeurs respectives d'une pluralité de facteurs de risque de maladie pour le sujet ;
b) fournir une base de données d'individus pour lesquels les valeurs de la pluralité de facteurs de risque de maladie ont été déterminées et pour lesquels l'apparition ou non de la maladie chez les individus est connue ;
c) transformer chaque valeur de la pluralité de facteurs de risque de maladie du sujet et des individus de la base de données sur une même échelle d'incidence de la maladie, dans laquelle chaque valeur de facteur de risque de maladie est transformée par l'incidence de la maladie dans une population d'individus ayant la même valeur de facteur de risque de maladie :
d) sélectionner, dans la base de données, un nombre d'individus qui se situent à la plus faible distance euclidienne du sujet par rapport aux autres individus de la base de données, dans laquelle la distance euclidienne est basée sur les valeurs transformées de la pluralité de facteurs de risque de maladie et dans laquelle le nombre d'individus qui se situent à la plus faible distance euclidienne du sujet est entre 10 individus et 10 000 individus :

e) déterminer le rapport de la quantité d'individus sélectionnés chez lesquels la maladie s'est déclarée sur le nombre d'individus sélectionnés, pronostiquer ainsi le risque d'apparition de la maladie chez le sujet ;

dans laquelle la méthode est une méthode mise en oeuvre par ordinateur.

2. Méthode selon la revendication 1, dans laquelle au moins un facteur de risque de maladie est qualitatif.

3. Méthode selon l'une des revendications 1 ou 2, dans laquelle le nombre d'individus qui se situent à la plus faible distance euclidienne du sujet est tel que la quantité d'individus sélectionnés chez qui la maladie est apparue est d'au moins 10.

4. Méthode selon l'une des revendications 1 à 3, dans laquelle la maladie est sélectionnée dans la liste constituée d'un cancer, d'une maladie neurodégénérative, d'un événement cardiovasculaire, d'une maladie auto-immune, d'une allergie, d'un trouble endocrinien, et de l'ostéoporose.

5. Méthode selon l'une des revendications 1 à 4, dans laquelle la maladie est le cancer du sein.

6. Méthode selon l'une des revendications 1 à 5, dans laquelle au moins un facteur de risque de maladie est sélectionné dans le groupe constitué de l'âge, de la masse corporelle, de la taille, de l'indice de masse corporelle, d'une dimension corporelle, de l'apparition de la maladie chez un parent, de l'apparition d'une biopsie, de l'apparition ou du niveau d'un marqueur biochimique, de l'apparition ou du niveau d'un marqueur d'imagerie, de l'apparition ou du niveau d'un marqueur génétique, du lieu de vie ou de travail, de la race ou de l'origine ethnique, du niveau d'exercice physique

7. Méthode selon l'une des revendications 1 à 6, dans laquelle la maladie est le cancer du sein et au moins un facteur de risque de maladie est sélectionné dans le groupe constitué par le sexe, l'âge, l'apparition d'un cancer du sein chez un parent, l'apparition ou le niveau d'un marqueur génétique, la réalisation d'une biopsie du sein, l'apparition antérieure d'un cancer du sein, la survenue de radiothérapie de la poitrine ou du visage, l'apparition d'une affection bénigne du sein, la race ou l'origine ethnique, l'indice de masse corporelle, les antécédents de grossesse, les antécédents d'allaitement, les antécédents menstruels, l'utilisation d'une thérapie hormonale de remplacement, le niveau de consommation d'alcool, la densité mammaire, le niveau d'exercice physique, le niveau de tabagisme, le niveau de vitamine D, le niveau d'exposition à la lumière la nuit, le niveau d'exposition au diéthylstilbestrol (DES), le niveau de consommation d'aliments malsains, le niveau de consommation d'aliments grillés, cuits au barbecue ou fumés, le niveau d'exposition aux produits chimiques dans les cosmétiques, le niveau d'exposition aux produits chimiques dans les aliments, le niveau d'exposition aux produits chimiques pour la pelouse et le jardin, le niveau d'exposition aux produits chimiques dans le plastique, le niveau d'exposition aux produits chimiques dans la crème solaire, et le niveau d'exposition aux produits chimiques dans l'eau.

8. Méthode selon l'une des revendications 1 à 7, dans laquelle la maladie est le cancer du sein et au moins un facteur de risque de maladie est sélectionné dans le groupe constitué de l'âge, de l'apparition d'un cancer du sein chez un parent, de la réalisation d'une biopsie mammaire, de la race ou de l'origine ethnique et de la densité mammaire.

9. Méthode selon l'une des revendications 1 à 8, dans laquelle la maladie est le cancer du sein et la pluralité de facteurs de risque de maladie comprend au moins l'âge, l'apparition d'un cancer du sein chez un parent, la réalisation d'une biopsie du sein, la race ou l'origine ethnique, et la densité mammaire.

10. Méthode selon l'une des revendications 7 à 9, dans laquelle la densité mammaire est évaluée à l'aide du système BI-RADS (Breast Imaging-Reporting and Data System).

11. Méthode de détermination d'une valeur estimée d'un facteur biologique chez un sujet pour lequel les valeurs respectives d'une pluralité de facteurs cliniques ont été déterminées, comprenant :

a) fournir une base de données d'individus pour lesquels les valeurs de la pluralité de facteurs cliniques et du facteur biologique ont été déterminées et l'apparition ou non d'une maladie chez les individus est connue :

b) transformer chaque valeur de la pluralité de facteurs cliniques du sujet et des individus de la base de données sur une même échelle d'incidence de la maladie dans laquelle chaque valeur de facteurs cliniques est transformée par l'incidence de la maladie dans une population d'individus ayant la même dite valeur de facteur clinique ;

c) sélectionner, dans la base de données, un nombre d'individus qui se situent à la plus faible distance euclidienne du sujet par rapport aux autres individus de la base de données, dans laquelle la distance euclidienne est basée sur les valeurs transformées de la pluralité de facteurs cliniques et dans laquelle le nombre d'individus qui se situent à la plus faible distance euclidienne du sujet est entre 10 individus et 10 000 individus :

d) déterminer la valeur moyenne ou médiane du facteur biologique des individus sélectionnés, obtenir ainsi la valeur estimée du facteur biologique du sujet.

**Figure 1**

**Figure 2**

Figure 3

Figure 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **GAIL et al.** *J Natl Cancer Inst,* 1989, vol. 81, 1879-1886
- **COSTANTINO et al.** *J Natl Cancer Inst,* 1999, vol. 91, 1541-1548
- **TYRER et al.** *Stat Med,* 2004, vol. 23, 1111-1130
- **TICE et al.** *Breast Cancer Res Treat,* 2005, vol. 94, 115-122
- **BARLOW et al.** *J Natl Cancer Inst,* 2006, vol. 98, 1204-1214
- **CHEN et al.** *J Natl Cancer Inst,* 2006, vol. 98, 1215-1226
- **TICE et al.** *Ann Intern Med,* 2008, vol. 148, 337-347
- **GAUTHIER et al.** *The international conference on data mining:,* 2011, vol. 15-21
- **TICE et al.** *Ann. Intern. Med.,* 2008, vol. 148, 37-347, https://tools.bcsc-scc.org/BC5yearRisk/calculator.htm
- **RESTON, VA.** Breast Imaging Reporting and Data System Atlas. American College of Radiology, 2003
- **FIX ; HODGES ; RANDOLPH FIELD, TX ; COVER ; HART.** Discriminatory analysis, non parametric discrimination: consistency properties. *IEEE Transactions on Information Theory,* 1951, vol. 13, 21-27
- **EGAN.** Signal detection theory and ROC analysis. Cognition and Perception Academic Press, 1975
- **VARGHESE ; EASTON.** *Curr Opin Genet Dev,* 2010, vol. 20, 201-209
- **MICHAILIDOU et al.** *Nat Genet,* 2013, vol. 45, 353-361
- **SAKODA et al.** *Nat Genet,* 2013, vol. 45, 345-348
- **PASHAYAN et al.** *Nat Genet,* 2011, vol. 45, 345-348
- **EVANS et al.** *Cancer Prev Res,* 2012, vol. 5, 943-951
- **ALTSCHUL et al.** *Nat Genet,* 1990, vol. 45, 345-348
- **ZHENG et al.** *Hum Mol Genet,* 2013, vol. 22, 2539-2550
- **HILBERS et al.** *Clin Genet,* 2013, vol. 84, 407-414
- **BRENNAN et al.** *Cancer Res,* 2012, vol. 72, 2304-2313
- **XU et al.** *J Natl Cancer Inst,* 2013, vol. 105, 694-700
- **DENKERT et al.** *Genome Med,* 2012, vol. 4, 37